# EUROPEAN PATENT SPECIFICATION

(11) **EP 3 964 196 B1**
(45) Date of publication and mention of the grant of the patent: **21.10.2026**
(21) Application number: 20209503.0
(22) Date of filing: 24.11.2020
(51) Int. Cl.: A61K 9/00, A61K 9/08

(54) **USE OF CHLORHEXIDINE AND CETYLPYRIDINIUM CHLORIDE AS ANTIVIRAL AGENTS**
VERWENDUNG VON CHLORHEXIDIN UND CETYLPYRIDINIUMCHLORID ALS ANTIVIRALE MITTEL
UTILISATION DE CHLORHEXIDINE ET DE CHLORURE DE CÉTYLPYRIDINIUM EN TANT QU'AGENTS ANTIVIRAUX

(43) Date of publication of application: 09.03.2022
(73) Proprietor: Ferton Holding S.A., 2800 Delémont (CH)
(72) Inventor: LUSSI, Adrian, 3076 Worb (CH); ANTON, Oliver Martin, 68647 Biblis (DE); WITTMANN, Jörg, 64285 Darmstadt (DE)
(74) Representative: Müller Schupfner & Partner Patent- und Rechtsanwaltspartnerschaft mbB (Muc)

(56) References cited:
- EP-A2- 0 920 857
- US-A1- 2005 100 601
- MOOSAVI MAHDIEH-SADAT ET AL: "Antiviral mouthwashes: possible benefit for COVID-19 with evidence-based approach", JOURNAL OF ORAL MICROBIOLOGY, vol. 12, no. 1, 1 January 2020 (2020-01-01), pages 1794363, XP055828110, Retrieved from the Internet <URL:http://socendoconcepcion.cl/descargas/covid19/articulos/Antiviral%20mouthwashes%20possible%20benefit%20for%20COVID%2019%20with%20evidence%20based%20approach.pdf> DOI: 10.1080/20002297.2020.1794363

## Description

The invention relates to a composition comprising chlorhexidine, cetylpyridinium chloride and a surface active agent for use as antiviral agent in the oral cavity against SARS-CoV-2.

Antibacterial agents such as chlorhexidine, cetylpyridinium chloride and triclosan have been recognized as useful for treating oral health conditions such as plaque, caries, periodontal diseases and halitosis. The effectiveness of these compounds is due to their antibacterial activity. They have been used in commercial mouthwash products and several other types of oral care products.

WO 03/002056 A1 discloses an oral composition comprising an antibacterial effective amount of cetylpyridinium chloride and additional compounds as well as an orally acceptable carrier.

US 2005/0100601 A1 discloses a method for inactivating viral pathogens.

Moosavi Mahdieh-Sadat et al. "Antiviral mouthwashes: possible benefit for COVID-19 with evidence-based approach", Journal of Oral Microbiology, vol. 12, no. 1, 1 January 2020, page 1794363, XP055828110, discloses antiviral mouthwashes and possible benefits for COVID-19.

In the course of spread of SARS-CoV-2, resulting in the Covid-19 pandemic, it has been recognized that the reduction of viruses also in the oral cavity may help to diminish the infection rate with SARS-CoV-2. Chlorhexidine and cetylpyridinium chloride are both active against bacteria, but there is still need for an effective virucidal composition.

In view of the above, it is an object of the present invention to provide a composition that is useful as antiviral agent in the oral cavity. It is a further object of the present invention to provide a process for reducing the number of active viruses in the oral cavity.

This object is achieved by a composition comprising
a) 0.01 - 0.3 wt.-% chlorhexidine or an orally acceptable salt thereof, based on the total weight of the composition,
b) 0.02 - 0.2 wt.-% cetylpyridinium chloride, based on the total weight of the composition, and
c) 0.1 to 10 wt.-% of at least one surface active agent in water

for use as antiviral agent against SARS-CoV-2 in the oral cavity, wherein
the at least one surface active agent is a non-ionic surface active agent which is a glycerol containing compound
and/or polyethylene glycol containing compound.

A process for reducing the number of active viruses in the oral cavity is also described, comprising administering to the oral cavity an effective amount of this composition.

Preferred embodiments of the invention are subject to the dependent claims as well as the following description.

It has surprisingly been found that the combination of chlorhexidine, cetylpyridinium chloride and a surface active agent in aqueous solution results in a much higher virucidal activity against SARS-CoV-2 than chlorhexidine or cetylpyridinium chloride alone or a combination of chlorhexidine and cetylpyridinium chloride, but without surface active agent being present.

Without being bounded to this theory, it is believed that the surface active agent helps to destroy and thereby at least partially opening the virus shell so that chlorhexidine and cetylpyridinium chloride can be more effective in attacking viruses.

"Destroying" a virus, "inactivating" a virus or "reducing the number of active virus", in particular SARS-CoV-2, within the meaning of the invention is the reduction of the number of active viruses by applying the composition of the invention to such virus. This reduction of the number of active viruses can be detected with a standard plaque assay as described in the experimental section of the present application.

The composition according to the present invention is for use in the oral cavity. Therefore, the composition can also be referred to as an oral composition. The oral cavity can be the oral cavity of humans or animals, wherein the oral cavity of humans is preferred.

The oral composition contains the three components chlorhexidine, cetylpyridinium chloride and a surface active agent in water, preferably it is a solution in water. The composition may additionally contain ethanol, preferably in an amount of 3 - 25 wt.- % (weight percent), more preferably 5 - 15 wt.-% and most preferably 8 - 12 wt.-%.

The amount of chlorhexidine in the composition of the present invention is 0.01 - 0.3 wt.-%, preferably 0.05 - 0.15 wt.-%, more preferably 0.08 - 0.12 wt.-% and most preferably 0.1 wt.-% chlorhexidine. The aforementioned amounts are based on the total weight of the composition. By way of example, 0.1 wt.-% chlorhexidine means an amount that corresponds to 0.1 g chlorhexidine in 100 g of the composition.

The chlorhexidine of the present invention can be added as such or as an orally acceptable salt thereof. Preferred salts are chlorhexidine gluconates, chlorides or acetates, in particular chlorhexidine digluconate, chlorhexidine dichloride or chlorhexidine diacetate. The chlorhexidine salt is most preferably chlorhexidine digluconate. If an orally acceptable salt is contained in the composition of the invention, for example chlorhexidine digluconate, the above-mentioned amounts (for example 0.01 - 0.3 wt.-%) are still calculated in relation to chlorhexidine, i.e. not calculated in relation to the salt. By way of example, if the composition contains 0.1 wt.-% chlorhexidine, wherein the chlorhexidine is chlorhexidine digluconate, this means the composition contains 0.1 g chlorhexidine in 100 g of the composition.

The amount of cetylpyridinium chloride in the composition according to the invention is preferably 0.01 - 0.08 wt.-%, more preferably 0.03 - 0.07 wt.-% and most preferably 0.04 - 0.06 wt.-% cetylpyridinium chloride, based on the total weight of the composition.

The surface active agent (surfactant) enhances the effectiveness of the two antiviral agents, chlorhexidine and cetylpyridinium chloride. The surface active agent is non-ionic. Non-ionic surface active agents usually do not significantly influence the effectiveness of the chlorhexidine. The surface active agent is a non-ionic surfactant, specifically a glycerol containing compound and/or polyethylene glycol containing compound, preferably a polyethylene glycol derivative of hydrogenated castor oil, in particular PEG-20 hydrogenated castor oil or PEG-40 hydrogenated castor oil. The amount of the surfactant in the composition of the present invention is 0.1 - 1.0 wt.- %, preferably 0.2 - 0.8 wt.-%, more preferably 0.2 - 0.5 wt.-%, based on the total weight of the composition.

In a preferred embodiment of the invention, the composition further comprises a sweetener and/or an alditol, in particular for improving the taste of the composition. The sweetener is preferably selected from the group consisting of acesulfame, aspartame, cyclamate, saccharin, sucralose and mixtures thereof. Saccharin, cyclamate, sucralose or a mixture thereof are more preferred, sucralose being most preferred. The alditol is preferably selected from the group consisting of erythritol, sorbitol, xylite (xylitol), mannit (mannitol), lactitol, threit (threitol), arabitol, maltitol, isomalt and mixtures thereof. Xylite, sorbitol and mixtures thereof are most preferred.

The one or more alditol and/or the one or more sweetener are each preferably contained in the composition of the present invention in an amount of 0.01 - 1 wt.-%, more preferably 0.02 - 0.5 wt.-%, based on the total weight of the composition.

In a further preferred embodiment of the invention, the composition may additionally contain a polyol or a mixture of polyols. These polyols include in particular glycerol (glycerine), propylene glycol and polyethylene glycol. Among the polyethylene glycols, those with a molecular weight between 300 and 5.000 are preferred. The polyol or mixture of polyols is preferably contained in the composition of the present invention in an amount of 0.01 - 1 wt.-%, more preferably 0.02 - 0.5 wt.-%, based on the total weight of the composition.

The composition according to the invention preferably comprises additionally one or more essential oils. These essential oils can be added to further improve the taste and/or odor of the composition. In principle, all essential oils that have already been used in mouth or throat rinsing solutions can be used. The essential oils are preferably selected from the group consisting of peppermint oil, eucalyptus oil, lemon oil, orange oil, thyme oil, chamomile oil and mixtures thereof. Among these, peppermint oil and eucalyptus oil or mixtures thereof are most preferred. Suitable quantities of the essential oils are 0.2 - 4 wt.-%, preferably 0.5 - 3 wt.-%, based on the total weight of the composition.

It is further preferred that the composition comprises a fluoride, for example sodium fluoride.

According to a preferred embodiment of the invention, the composition for use as antiviral agent in the oral cavity comprises
a) 0.08 - 0.12 wt.-% chlorhexidine digluconate,
b) 0.03 - 0.07 wt.-% cetylpyridinium chloride,
c) 0.1 - 1.0 wt.-% PEG-40 hydrogenated castor oil,
d) glycerol,
e) propylene glycol,
f) sucralose,
g) xylite,
h) a fluoride and
i) water.

The above amounts are based on the total weight of the composition. The above composition may further comprise ethanol, preferably 3 - 25 wt.-% ethanol, more preferably 5 - 15 wt.-% ethanol, based on the total weight of the composition.

The composition according to the present invention is an aqueous solution for use as mouthwash, for use in a mouthwash device or for use in a powder jet device for tooth cleaning. The use in a mouthwash device may be a use in commercial mouthwash devices for consumers. For using the composition of the invention in a powder jet device for tooth cleaning, the liquid composition can be added to the gaseous carrier medium which is sprayed together with a powder onto tooth surfaces, thereby cleaning of the tooth surfaces by the powder and inactivating viruses on the tooth surfaces and also in possible aerosols that may be formed during the powder jet cleaning process.

The invention further relates to a process for reducing the number of active viruses in the oral cavity of humans or animals, comprising administering to the oral cavity an effective amount of the composition that has been described above. Administering the composition to the oral cavity can be in the form of using a mouthwash or a mouthwash device or the use of the composition in a powder jet device as described above.

The following examples provide preferred embodiments according to the invention.

### Examples

A mouth rinsing solution according to the invention (see example 1 below) and some of their individual active components were tested regarding their virucidal efficacy against the novel coronavirus SARS-CoV-2, *in vitro.* To evaluate if this procedure is sufficient to successfully inactivate SARS-CoV-2, in the following study 1x10⁶ infectious SARS-CoV-2 particles were incubated with the solutions (dilution factor: 2) for 30sec. After a serial dilution the samples were submitted to a standard plaque assay to check for the presence of infectious virus particles post incubation with the different solutions.

### Abbreviations

| | |
|---|---|
| BSA | Bovine Serum Albumin |
| CHX | Chlorhexidine |
| CPC | Cetylpyridinium chloride |
| CPE | Cytopathic Effect |
| ddH₂O | Double distilled water |
| DEAE-Dextran | Diethylaminoethyl-dextran hydrochloride |
| Dil. | Dilution |
| h | Hour(s) |
| H₂O₂ | Hydrogen peroxide |
| IMDM | Iscove's Modified Dulbecco's Medium |
| Inf. | Infection |
| MEM | Minimum Essential Medium |
| min | Minute |
| mL | Milliliter |
| µL | Microliter |
| MRS | Mouth Rinsing Solution |
| NaHCO₃ | Sodium hydrogen carbonate |
| PBS | Phosphate Buffered Saline |
| pfu | Plaque Forming Units |
| SARS-CoV-2 | Severe acute respiratory syndrome coronavirus 2 |
| sec | Second(s) |

### Material and Methods

| | |
|---|---|
| Cells | Vero E6: Cercopithecus aethiops, Epithelial cells, Kidney, ATCC (American Type Culture Collection, Cat. No. CRL-1586) |
| Virus | Severe acute respiratory syndrome coronavirus 2 (SARS-CoV-2; Isolate "FI-100"; Westfaelische Wilhelms-University Muenster, Institute of Virology (IVM)) Stock titer: 1.25x10⁷ pfu/mL |
| Seeding density | 6-Well: 1 x 10⁶ cells per well, 2mL/well |
| Inoculation volume | 6-Well: 500µL |
| Culture volume | 6-Well: 2mL |
| Cultivation Conditions | Vero E6: 37°C, 5% CO₂, humidified incubator |
| Cell Culture Medium | Vero E6: IMDM medium (Gibco, Cat. No. 12440-053) containing 10% Fetal Bovine Serum (Capricorn Scientific GmbH, Cat. No. FBS-12A), 1% Penicillin/Streptomycin (Sigma-Aldrich Chemie GmbH, Cat. No. P4333) |
| Infection Medium | Vero E6: IMDM medium containing (Gibco, Cat. No. 12440-053), 5% Fetal Bovine Serum (Capricorn Scientific GmbH, Cat. No. FBS-12A), 1% Penicillin/Streptomycin (Sigma-Aldrich Chemie GmbH, Cat. No. P4333) |
| Avicel-Medium (Plaque Medium) | 1.25% Avicel (FMC BioPolymer, Cat. No. RC581), 10% MEM (Gibco, Cat. No. 21430-20), 0.01% DEAE-Dextran (Sigma Aldrich, Cat. No. D9885), 2.8% NaHCO3 (Merck, Cat. No. 1.06329.1000), 1% Penicillin/Streptomycin (Sigma Aldrich, Cat. No. P4333), 0.2% BSA (Carl Roth, Cat. No. 9163.4), 1% L-Glutamine (Sigma Aldrich, Cat. No. G7513) |
| 4% Roti-Histofix | 4% Roti-Histofix (Carl Roth, Cat. No. A146.1) in PBS (Lonza, Cat. No. 17515Q) |
| Crystal violet solution | 1% Crystal violet (Merck, Cat. No. 1408), 10% Ethanol (SAVIP, Cat. No. ETO-5000-99-1) in ddH₂O |

### Incubation of SARS-CoV-2 with Mouth Rinsing Solutions (MRS)

### Test solutions:

- Example 1 (0.1% Chlorhexidine (CHX), 0.05% CPC, PEG-40 hydrogenated castor oil), according to the invention

### Comparative examples:

- 0.1% CHX plus 0.05% CPC
- 0.05% CPC
- 0.1% CHX
- 1.5% H₂O₂
- 0.5% H₂O₂

For every solution tested, three samples were prepared in 1.5mL-reaction tubes:
a) "SARS-CoV-2 + MRS": Infectious SARS-CoV-2 particles (80µL ≙ 1x10⁶ pfu) + MRS (80µL)
b) "SARS-CoV-2 + Inf. Medium" (negative control): Infectious SARS-CoV-2 particles (80µL ≙ 1x10⁶ pfu) + Infection Medium (80µL)
c) "MRS + Inf. Medium" (positive control): MRS (80µL) + Infection Medium (80µL)

The samples were mixed briefly by vortexing and incubated at 37°C for 30sec. Immediately after the incubation, the samples were diluted by addition of 1440µL Infection Medium followed by a 10-fold serial dilution and one 20-fold dilution step as described in Table 1. Dilution 4 of each sample was analyzed in technical duplicates in a plaque assay.

The serial dilution was conducted to avoid any cytotoxic effects of the MRS in the plaque assay.

**Table 1 Serial dilution of samples for analysis in plaque assay**

| | Dilution 1 | Dilution 2 | Dilution 3 | Dilution 4 |
|---|---|---|---|---|
| Dilution factor | 10 | 10 | 10 | 20 |
| Sample / **Dilution** | 160µL Sample | 160µL Dilution 1 | 160µL Dilution 2 | 80µL Dilution 3 |
| Infection Medium | 1440 µL | 1440µL | 1440µL | 1520µL |
| Dil. Factor MRS | 20 | 200 | 2000 | 40 000 |

### Plaque Assay

To test the samples for the presence of infectious SARS-CoV-2 virus particles, a standard plaque assay with Avicel overlay was performed. Vero E6 cells were seeded at 1x10⁶ cells/well in 6-well plates and incubated for 24h, 37°C, 5% CO₂. The next day the cells were washed once with Infection Medium before inoculation with 500µL of the prediluted samples per well. Incubation for 1h at 37°C, 5% CO₂. Afterwards the inoculum was removed completely, and the cells were overlaid with Avicel-Medium. Incubation at 37°C, 5% CO₂. After 72h the Avicel-Medium was removed. The cells were rinsed twice with PBS, fixed with 4% Roti-Histofix for 30min at 4°C and stained with Crystal violet. Afterwards the plates were left to dry. Photos were taken for record.

### Results

The virucidal activity of example 1 and some of their individual components were investigated against the novel corona virus SARS-CoV-2 by a 30sec incubation of 1x10⁶ pfu SARS-CoV-2 with the test substances, followed by a standard plaque assay. CPE (cytopathic effect)-inducing viruses like SARS-CoV-2 form plaques (areas in which the cell layer is destroyed by the virus infection) in a plaque assay. Each plaque in a well represents one infectious virus particle in the sample. Crystal violet stains cells, therefore, the intact cell layer appears violet, while plaques are visible as white circular areas. All plates follow the same layout with the "SARS-CoV-2 + MRS" sample in column 1, the "SARS-CoV-2 + Inf. Medium", negative control, in column 2 and the "MRS + Inf. Medium", positive control, in column 3, each analyzed in technical duplicates.

The negative control "SARS-CoV-2 + Inf. Medium" showed plaques for all tested solutions, as expected and the positive controls "MRS + Inf. Medium" showed an intact cell layer in all set-ups, demonstrating, that the test substance had no cytotoxic effect in the dilution (1:40,000) used in the plaque assay. Small areas with cell loss at the edges of the wells come from the pipetting process and are not caused by viral activity.

For example 1, no infectious SARS-CoV-2 particles were detected in the plaque assay. In contrast, plaques were detected for their individual compounds (the above comparative examples) after 30sec incubation of the virus with the solutions. The combination of 0.1% CHX + 0.05%CPC and 0.05% CPC alone showed some reduction in the number of plaques. 0.1% CHX, 1.5% H₂O₂, and 0.5% H₂O₂ alone showed no reduction compared to the negative control.

### Summary of the Examples

The above results demonstrate the virucidal efficacy of the mouth rinsing solution according to example 1 *in vitro.* No infectious SARS-CoV-2 particles were detectable after incubation of 1x10⁶ infectious SARS-CoV-2 particles with the solution (2-fold diluted) for 30sec followed by a serial dilution and a standard plaque assay. In contrast, their individual components did not inactivate the virus completely.

## Claims

1. Composition comprising
a) 0.01 - 0.3 wt.-% chlorhexidine or an orally acceptable salt thereof, based on the total weight of the composition,
b) 0.02 - 0.2 wt.-% cetylpyridinium chloride, based on the total weight of the composition, and
c) 0.1 - 1.0 wt.-% of at least one surface active agent in water for use as antiviral agent against SARS-CoV-2 in the oral cavity, **characterized in that** the at least one surface active agent is a non-ionic surface active agent which is a glycerol containing compound and/or polyethylene glycol containing compound.

2. Composition for use according to claim 1, **characterized in that** the composition comprises
a) 0.08 - 0.12 wt.-% chlorhexidine or an orally acceptable salt thereof, based on the total weight of the composition, and
b) 0.03 - 0.07 wt.-% cetylpyridinium chloride, based on the total weight of the composition.

3. Composition for use according to claim 1 or 2, **characterized in that** the chlorhexidine or an orally acceptable salt thereof is chlorhexidine digluconate.

4. Composition for use according to any of claims 1 to 3, **characterized in that** the composition comprises 0.2 - 0.5 wt.-% of the surface active agent, based on the total weight of the composition.

5. Composition for use according to any of claims 1 to 4, **characterized in that** the surface active agent is a polyethylene glycol derivative of hydrogenated castor oil, preferably PEG-40 hydrogenated castor oil.

6. Composition for use according to any of claims 1 to 5, **characterized in that** the composition further comprises 3 - 25 wt.-% ethanol, based on the total weight of the composition.

7. Composition for use according to any of claims 1 to 6, **characterized in that** the composition further comprises one or more selected from the group consisting of sweeteners, alditols, polyols, essential oils and fluorides.

8. Composition for use according to claim 7, **characterized in that** the sweetener is sucralose and/or the alditol is xylite.

9. Composition for use according to claim 7 or 8, **characterized in that** the essential oil is peppermint oil, eucalyptus oil or a mixture thereof.

10. Composition for use according to any of claims 7 to 9, **characterized in that** the polyol is glycerol, propylene glycol or a mixture thereof.

11. Composition for use according to any of claims 1 to 10, **characterized in that** the composition comprises at least one additional antibacterial agent, preferably H₂O₂.

12. Composition for use according to any of claims 1 to 11, **characterized in that** the composition is used as mouth rinse, in a mouthwash device or in a powder jet device for tooth cleaning.

## Patentansprüche

1. Zusammensetzung, umfassend
a) 0,01 - 0,3 Gew.-% Chlorhexidin oder ein oral verträgliches Salz davon, bezogen auf das Gesamtgewicht der Zusammensetzung,
b) 0,02 - 0,2 Gew.-% Cetylpyridiniumchlorid, bezogen auf das Gesamtgewicht der Zusammensetzung, und
c) 0,1 - 1,0 Gew.-% zumindest eines Tensids in Wasser zur Verwendung als antivirales Mittel gegen SARS-CoV-2 in der Mundhöhle, **dadurch gekennzeichnet, dass** das zumindest eine Tensid ein nichtionisches Tensid ist, bei dem es sich um eine glycerinhaltige Verbindung und/oder eine polyethylenglykolhaltige Verbindung handelt.

2. Zusammensetzung zur Verwendung gemäß Anspruch 1, **dadurch gekennzeichnet, dass** die Zusammensetzung umfasst
a) 0,08 - 0,12 Gew.-% Chlorhexidin oder ein oral verträgliches Salz davon, bezogen auf das Gesamtgewicht der Zusammensetzung, und
b) 0,03 - 0,07 Gew.-% Cetylpyridiniumchlorid, bezogen auf das Gesamtgewicht der Zusammensetzung.

3. Zusammensetzung zur Verwendung gemäß Anspruch 1 oder 2, **dadurch gekennzeichnet, dass** das Chlorhexidin oder ein oral verträgliches Salz davon Chlorhexidindigluconat ist.

4. Zusammensetzung zur Verwendung gemäß einem der Ansprüche 1 bis 3, **dadurch gekennzeichnet, dass** die Zusammensetzung 0,2 bis 0,5 Gew.-% des Tensids, bezogen auf das Gesamtgewicht der Zusammensetzung, umfasst.

5. Zusammensetzung zur Verwendung gemäß einem der Ansprüche 1 bis 4, **dadurch gekennzeichnet, dass** das Tensid ein Polyethylenglykol-Derivat von hydriertem Rizinusöl ist, vorzugsweise PEG-40-hydriertes Rizinusöl.

6. Zusammensetzung zur Verwendung gemäß einem der Ansprüche 1 bis 5, **dadurch gekennzeichnet, dass** die Zusammensetzung weiter 3 bis 25 Gew.-% Ethanol, bezogen auf das Gesamtgewicht der Zusammensetzung, umfasst.

7. Zusammensetzung zur Verwendung gemäß einem der Ansprüche 1 bis 6, **dadurch gekennzeichnet, dass** die Zusammensetzung weiter einen oder mehrere Stoffe umfasst, die aus der Gruppe ausgewählt sind, die aus Süßungsmitteln, Alditolen, Polyolen, ätherischen Ölen und Fluoriden besteht.

8. Zusammensetzung zur Verwendung gemäß Anspruch 7, **dadurch gekennzeichnet, dass** der Süßstoff Sucralose ist und/oder das Aldit xylit ist.

9. Zusammensetzung zur Verwendung gemäß Anspruch 7 oder 8, **dadurch gekennzeichnet, dass** es sich bei dem ätherischen Öl um Pfefferminzöl, Eukalyptusöl oder eine Mischung daraus handelt.

10. Zusammensetzung zur Verwendung gemäß einem der Ansprüche 7 bis 9, **dadurch gekennzeichnet, dass** das Polyol Glycerin, Propylenglykol oder eine Mischung daraus ist.

11. Zusammensetzung zur Verwendung gemäß einem der Ansprüche 1 bis 10,
**dadurch gekennzeichnet, dass** die Zusammensetzung mindestens einen zusätzlichen antibakteriellen Wirkstoff, vorzugsweise H₂O₂, umfasst.

12. Zusammensetzung zur Verwendung gemäß einem der Ansprüche 1 bis 11,
**dadurch gekennzeichnet, dass** die Zusammensetzung als Mundspülung, in einer Mundspülvorrichtung oder in einem Pulverstrahlgerät zur Zahnreinigung verwendet wird.

## Revendications

1. Composition comprenant
a) 0,01 à 0,3 % en poids de chlorhexidine ou d'un de ses sels acceptables par voie orale, par rapport au poids total de la composition,
b) 0,02 à 0,2 % en poids de chlorure de cétylpyridinium, par rapport au poids total de la composition, et
c) 0,1 à 1,0 % en poids d'au moins un agent tensioactif en solution aqueuse, destiné à être utilisé comme agent antiviral contre le SARS-CoV-2 dans la cavité buccale,
**caractérisée en ce que** ledit au moins un agent tensioactif est un agent tensioactif non ionique qui est un composé contenant du glycérol et/ou un composé contenant du polyéthylène glycol.

2. Composition destinée à être utilisée selon la revendication 1,
**caractérisée en ce que** la composition comprend
a) 0,08 à 0,12 % en poids de chlorhexidine ou d'un de ses sels acceptables par voie orale, par rapport au poids total de la composition, et
b) 0,03 à 0,07 % en poids de chlorure de cétylpyridinium, par rapport au poids total de la composition.

3. Composition destinée à être utilisée selon la revendication 1 ou 2,
**caractérisée en ce que** la chlorhexidine ou un de ses sels acceptables par voie orale est le digluconate de chlorhexidine.

4. Composition destinée à être utilisée selon l'une des revendications 1 à 3,
**caractérisée en ce que** la composition comprend 0,2 à 0,5 % en poids d'agent tensioactif, par rapport au poids total de la composition.

5. Composition destinée à être utilisée selon l'une des revendications 1 à 4,
**caractérisée en ce que** l'agent tensioactif est un dérivé de polyéthylène glycol de l'huile de ricin hydrogénée, de préférence de l'huile de ricin hydrogénée PEG-40.

6. Composition destinée à être utilisée selon l'une des revendications 1 à 5,
**caractérisée en ce que** la composition comprend en outre 3 à 25 % en poids d'éthanol, par rapport au poids total de la composition.

7. Composition destinée à être utilisée selon l'une des revendications 1 à 6,
**caractérisée en ce que** la composition comprend en outre un ou plusieurs éléments choisis dans le groupe constitué par les édulcorants, les alditols, les polyols, les huiles essentielles et les fluorures.

8. Composition destinée à être utilisée selon la revendication 7,
**caractérisée en ce que** l'édulcorant est le sucralose et/ou l'alditol est le xylitol.

9. Composition destinée à être utilisée selon la revendication 7 ou 8,
**caractérisée en ce que** l'huile essentielle est de l'huile de menthe poivrée, de l'huile d'eucalyptus ou un mélange de celles-ci.

10. Composition destinée à être utilisée selon l'une des revendications 7 à 9,
**caractérisée en ce que** le polyol est du glycérol, du propylène glycol ou un mélange de ceux-ci.

11. Composition destinée à être utilisée selon l'une des revendications 1 à 10,
**caractérisée en ce que** la composition comprend au moins un agent antibactérien supplémentaire, de préférence du H₂O₂.

12. Composition destinée à être utilisée selon l'une des revendications 1 à 11,
**caractérisée en ce que** la composition est utilisée comme bain de bouche, dans un dispositif de bain de bouche ou dans un dispositif à jet de poudre pour le nettoyage des dents.
